(19) Europäisches Patentamt
European Patent Office
Office européen des brevets

(11) EP 1 001 267 A2

(12) EUROPEAN PATENT APPLICATION

(43) Date of publication:
17.05.2000 Bulletin 2000/20

(51) Int. Cl.[7]: G01N 33/545, C12Q 1/68

(21) Application number: 99308958.0

(22) Date of filing: 10.11.1999

(84) Designated Contracting States:
AT BE CH CY DE DK ES FI FR GB GR IE IT LI LU MC NL PT SE
Designated Extension States:
AL LT LV MK RO SI

(30) Priority: 10.11.1998 JP 31892498
10.11.1998 JP 31892598

(71) Applicant:
NISSHINBO INDUSTRIES, INC.
Chuo-ku, Tokyo 103 (JP)

(72) Inventors:
- Suzuki, Osamu,
  c/o Nisshinbo Industries, Inc.
  Adachi-ku, Tokyo (JP)
- Shiohata, Namiko,
  c/o Nisshinbo Industries, Inc.
  Adachi-ku, Tokyo (JP)
- Matsumura, Yoshiyuki,
  c/o Nisshinbo Industries Co.
  Adachi-ku, Tokyo (JP)

(74) Representative:
Bannerman, David Gardner et al
Withers & Rogers,
Goldings House,
2 Hays Lane
London SE1 2HW (GB)

# (54) Carrier for immobilizing biologically active substance

(57) A base material having an isocyanate group or a carbodiimide group on its surface is used as a carrier for immobilizing a biologically active substance to analyze a first biologically active substance or a second biologically active substance in a sample, wherein the first substance immobilized on the carrier is reacted with the second substance capable of specifically binding to the first substance, and the second substance indirectly bound to the carrier by the bond between it and the first substance or the second substance that is bound to the carrier is detected.

EP 1 001 267 A2

## Description

BACKGROUND OF THE INVENTION

The present invention relates to a carrier for immobilizing a biologically active substance such as nucleic acid, an antibody, or an antigen, a method for producing the carrier, a method for immobilizing a biologically active substance on the carrier, and an assay method using the carrier.

In the field of clinical test, inspection of food, medicolegal examination, etc. a biologically active substance such as nucleic acid, an antibody, or an antigen is identified by the nucleic acid probing method, enzyme immunoassay, or similar methods, depending on a target substance.

Detection of nucleic acid is carried out in identification of species of pathological microorganisms, DNA identification for medicolegal purpose, and the like fields. To detect nucleic acid, nucleic acid having a nucleotide sequence complementary to that of a target nucleic acid is labeled directly with an enzyme or indirectly via a hapten, etc, and the labeled nucleic acid is allowed to hybridize with the target nucleic acid. After the labeled nucleic acid that is not hybridized with the target nucleic acid is removed or the label is inactivated, the label is detected to determine the presence or absence of the target nucleic acid and the amount of it.

Detection of an antigen or an antibody is carried out in the field of various clinical tests as well as identification of species of pathological microorganisms used for detecting nucleic acid. Competitive immunoassay, which is one embodiment of enzyme immunoassay used for detecting an antigen or an antibody, is carried out as follows. An antigen or an antibody is immobilized on the surface of a solid phase such as polystyrene beads, a microtiter plate, or a tube. A predetermined amount of a sample solution is applied to the surface of the solid phase, and an antigen-enzyme complex or an antibody-enzyme complex is then added. When an antibody is immobilized on the surface of a solid phase, an antigen in a sample solution and an antigen-enzyme complex are competitively bound to the antibody immobilized on the surface of the solid phase. When an antigen is immobilized on the surface of a solid phase, an antigen in a sample solution and the antigen immobilized on the surface of the solid phase are competitively bound to the antibody-enzyme complex.

After incubation for a certain period of time, when an antibody is immobilized, the antigen that is not bound to the immobilized antibody and the antigen-enzyme complex are removed by washing. When an antigen is immobilized, the unreacted antigen remaining in the sample solution, and the unreacted antibody-enzyme complex, and the binding product of the antigen in the sample solution and the antibody-enzyme complex are removed by washing. In this occasion, a wash solution is filled in the solid phase, then removed, a fresh wash solution is added again and removed. This washing procedure is usually repeated several to about ten times. This procedure is generally called B/F separation, which is essential for the detection method based on enzyme immunoassay.

At the final step, a solution of a chromogenic substrate for the enzyme used to label the antibody or the antigen is added to the solid phase, and the substrate is allowed to react with the remaining enzyme to develop color. Enzymes usually used include peroxidase, β-galactosidase, alkaline phosphatase, and the like. A chromogenic substrate used should be suitable for the enzyme used. If a target antigen exists in a sample solution in a large quantity, the amount of the remaining antigen-enzyme complex or the remaining antibody-enzyme complex decreases, thereby detecting low color intensity. The color intensity is measured with a colorimeter.

Sandwich enzyme immunoassay, which is another embodiment of enzyme immunoassay, is carried out as follows. An antibody is immobilized on the surface of a solid phase, and a sample solution is added to the surface of the solid phase. After incubation for a certain period of time, the antigen that is not bound to the antibody immobilized on the surface of the solid phase is removed by washing, and a predetermined amount of an antibody-enzyme complex is added. After incubation for a certain period of time, the antibody-enzyme complex that is not bound to the antigen immobilized on the surface of the solid phase is washed away, and a chromogenic substrate is added to the surface of the solid phase to develop color. The color intensity is measured to determine the concentration of the antigen contained in the sample solution.

As described above, in the method for detecting nucleic acid, competitive enzyme immunoassay, sandwich enzyme immunoassay or the like conventional methods, it is very important to immobilize an antibody, an antigen, an enzyme, nucleic acid, or the like capture molecule on the surface of a solid phase such as a tube, a microtiter plate, a membrane filter, or beads. Various methods for immobilizing a biologically active substance have been proposed. For example, known methods for immobilizing a protein are as follows:

(1) Methods of chemically binding a protein to a base material using a crosslinking agent or a condensing agent, including the diazo method, the peptide method, the alkylation method, the method for binding a protein to a base material using a crosslinking agent, and the method for binding a protein to a base material based on Ugi reaction ("Immobilized Enzyme," ed. By Ichiro Chihata, Kodansha Scientific (1986), pages 9-41);
(2) Methods for immobilizing a protein on a base material by an ionic bond ("Immobilized Enzyme," pages 41-43);

and

(3) Methods for immobilizing a protein on a base material by physical adsorption ("Immobilized Enzyme," pages 43-45).

The following methods are known as methods for immobilizing nucleic acid.

(1) Methods of chemically binding nucleic acid to which a modification residue is introduced, such as immobilization of nucleic acid having a thiol group at the 5' end on a bead-like base material containing thiol groups by a disulfide bond therebetween (P.J.R. Day, P.S. Flora, J.E. Fox, M.R. Walker, Biochem. J., 278, 735-740 (1991)) (The other methods pertaining to in this category are described in Soren, R.R., Mette, R.L., Svend, E.R., Anal. Biochem., 198, 138-142 (1991), Jonathan N.K., Joseph L.W., Joseph P.D., Rachel, E.M., Mary C., Eugene L.B., Nucleic Acids Res., 15, 2891-2909 (1987), Allan, J.M., Jeffrey, R.B., Terence, W.P., Biochem. J., 191, 855-858 (1980), J.A. Running, M. S. Urdea, Bio Techniques, 8, 276-279 (1990), etc.);

(2) Methods for immobilizing nucleic acid by physical adsorption such as immobilization of nucleic acid by adsorption on a nitrocellulose membrane or a nylon membrane by UV radiation or heat treatment (J. Sambrok, E.F. Fritsch, and T. Maniatis, Molecular Cloning, Cold Spring Harbor Laboratory Press, Second Edition, page 2.109-2.113 and page 9.34-9.46) or immobilization nucleic acid on a microtiter plate by physical adsorption (G.C.N. Parry and A.D.B. Malcolm, Biochem. Soc. Trans., 17, 230-231 (1989)); and

(3) Methods for immobilizing nucleic acid using a polycarbodiimide coated base material (Japanese Patent Application Laid-open No. Hei 8-23975 and No. Hei 8-334509).

It has been pointed out, however, that the conventional methods described above have problems. For example, the methods utilizing chemical binding require a specific reagent. In the case of immobilization via a peptide bond, an amino group has to be introduced to either an active substance or a base material, and a carboxyl group has to be introduced to the rest. Furthermore, introduced functional groups have to be condensed to each other using a condensing agent for immobilization. Thus, complicated operation is required.

In the chemical binding method, both of a base material and an active substance must have an amino group when a crosslinking agent, for example, glutaraldehyde, is used. In this case, since a base material itself is required to have a functional group, it is a key to select a suitable base material. However, it is difficult to select such a base material suitable for immobilization. Furthermore, a naturally occurring DNA and a synthetic DNA without a modification group, which contain only a functional group with poor reactivity (a terminal phosphate group, a terminal hydroxyl group, etc.) are difficult to be immobilized by the method utilizing chemical reactions. Thus, there are problems that an active substance without an active functional group cannot be immobilized.

The physical adsorption methods also have problems that the amount of immobilized product is influenced by absorbability of the base material, that the adsorbed active substance is likely to be detached, and that, when an active substance is a low molecular weight substance (oligomer), it is hardly adsorbed by the base material since it weakly interacts with the base material.

The method of coating the surface of a base material with polycarbodiimide is disadvantageous in that the coated film is detached depending on conditions to use the immobilized product due to the differences in the thermal expansion rate or friction between the base material and polycarbodiimide. This leads to a low yield of the immobilized product and varied detection results.

As discussed above, there remain many problems in immobilization techniques that are important to detect protein, nucleic acid, and the similar active substances.

## SUMMARY OF THE INVENTION

The present invention provides a carrier for immobilizing a biologically active substance simply, efficiently, and strongly, and a method for detecting a biologically active substance using the carrier.

As a result of earnest investigation to solve the above problems, the present inventors have found that, when a base material insoluble in solvents having an isocyanate group on its surface is used as a carrier, a biologically active substance can be immobilized simply and efficiently on the carrier by the isocyanate group on its surface, regardless of the shape of the carrier. The inventors have also found that the above-described carrier can be used to detect various biologically important substances with high sensitivity and high accuracy utilizing the active substance immobilized on the carrier. The present invention has thus been completed.

The present invention also provides a method for detecting a biologically active substance using a carrier having a carbodiimide group on which a biologically active substance can be immobilized simply, efficiently, and strongly.

The carrier for immobilizing a biologically active substance used in the present invention to achieve the

above objectives comprises a base material insoluble in solvents and a compound having a carbodiimide group bound to the surface of the base material via a covalent bond. The method for immobilizing a biologically active substance used in the present invention to achieve the above objectives comprises the step of contacting a biologically active substance that reacts with a carbodiimide group with a carrier comprising a base material insoluble in solvents and a compound having a carbodiimide group bound to the surface of the base material via a covalent bond.

Low molecular weight carbodiimide derivatives, such as dicyclohexylcarbodiimide and di-p-toluoylcarbodiimide have been widely used as a dehydration condensing agent for synthesizing esters and peptides. These carbodiimide derivatives readily form a carboxylic acid adduct as shown in the following reaction formula (formula (I)), and the adduct condenses with alcohol, amine, or carboxylic acid with releasing a urea derivative as a byproduct to form a corresponding to ester, amide, or acid anhydride (formula (II)). It was proposed to use these low molecular weight carbodiimide derivatives for immobilizing an active substance.

$$R'CO_2H+RN=C=NR \rightarrow R'C(=O)OC(NHR)=NR \quad (I)$$

$$R'C(=O)OC(NHR)=NR+R''OH \rightarrow R'C(=O)OR''+RNHCONHR \quad (II)$$

$$R''NH_2 \rightarrow R'C(=O)NHR''+RNHCONHR \quad (II')$$

$$R''CO_2H \rightarrow R'C(=O)OC(=O)R''+RNHCONHR \quad (II'')$$

However, these low molecular weight carbodiimide derivatives were originally developed as a condensing agent and were made to be soluble in a solvent. When these derivatives are bound to a base material, they are easily detached from the base material and are thus not practically usable. Polycarbodiimide containing a carbodiimide group in its molecule then was paid attention to. A technique to immobilize a biologically active substance was developed, which comprises coating polycarbodiimide dissolved in a solvent on an insoluble base material to form a film of polycarbodiimide on the surface of the base material and immobilizing various biologically active substances thereon (Japanese Patent Application Laid-open No. Hei 8-23975 and No. Hei 8-334509). However, this method to coat the surface of the base material with polycarbodiimide has disadvantages of a low yield of immobilization products, unstable assay results, and increase in background emission because the carbodiimide film detaches depending on conditions to use the immobilization products due to the differences in the thermal expansion rate or friction between the base material and polycarbodiimide.

The present inventors intensively investigated to solve various problems with respect to the above carrier containing a compound with a carbodiimide group. As a result, the inventors found that various biologically important substances can be detected with high sensitivity and high accuracy using an immobilized biologically active substance prepared by binding a compound with a carbodiimide group capable of strongly capturing a biologically active substance to the base material insoluble in solvents via a covalent bond, and immobilizing a biologically active substance thereon. The present invention was thus completed.

Specifically, the present invention relates to a carrier for immobilizing a biologically active substance comprising a base material insoluble in solvents having isocyanate groups or carbodiimide groups on its surface.

The present invention is related to a carrier for immobilizing a biologically active substance comprises a base material insoluble in solvents and a compound having an isocyanate group bound to the surface of the base material via a covalent bond.

Furthermore, the present invention is related to a carrier for immobilizing a biologically active substance comprises a base material insoluble in solvents and a compound having a carbodiimide group bound to the surface of the base material via a covalent bond.

The base material insoluble in solvents used for the carrier of the present invention is made of one or more than two materials selected from the group consisting of plastics, inorganic polymers, metals, natural polymers, and ceramics.

The present invention also provides a method of producing a carrier for immobilizing a biologically active substance comprising a base material insoluble in solvents having an isocyanate group on its surface, wherein said method comprises covalently binding a compound having (i) more than two isocyanate groups, (ii) more than one isocyanate groups and more than one functional groups other than an isocyanate group or (iii) more than one isocyanate groups and a halogen atom, to a functional group on the surface of the base material insoluble in solvents with leaving at least one free isocyanate group of said compound, said functional group is capable of covalently binding to an isocyanate group, a functional group other than an isocyanate group, or a halogen atom.

The present invention also provides a method of producing a carrier for immobilizing a biologically active substance comprising a base material insoluble in solvents and a compound having a carbodiimide group bound onto the surface of the base material via a covalent bond, wherein said method comprises covalently binding a compound

having (i) more than two carbodiimide groups or (ii) more than one carbodiimide groups and more than one functional groups other than a carbodiimide group, to a functional group on the surface of the base material insoluble in solvents with leaving at least one free carbodiimide group of said compound, said functional group is capable of covalently binding to a carbodiimide group, or a functional group other than a carbodiimide group.

Moreover, the present invention provides a method of immobilizing a biologically active substance which comprises contacting a biologically active substance that is reactive with an isocyanate group or a carbodiimide group with a carrier for immobilizing a biologically active substance comprising a base material insoluble in solvents and a compound having an isocyanate group or a carbodiimide group bound onto the surface of the base material via a covalent bond.

The biologically active substance used in the immobilization method of the present invention is one or more than two substances selected from the group consisting of proteins, peptides, substances that bind to antibodies, and nucleic acids.

Furthermore, the present invention provides a method for assaying a biologically active substance comprises reacting a first substance which is biologically active and immobilized on a carrier with a second substance capable of specifically binding to the first substance, and detecting the second substance which is indirectly bound to the carrier via a bond between itself and the first substance or is not bound, to analyze the first substance or the second substance in a sample, wherein said carrier is the one for immobilizing a biologically active substance comprising a base material insoluble in solvents and a compound having an isocyanate group or a carbodiimide group bound onto the surface of the base material via a covalent bond, and said first substance is immobilized on the carrier via the isocyanate group or the carbodiimide group.

The combination of the first substance and the second substance used in the assay method of the present invention includes the combination of nucleic acid as the first substance and another nucleic acid having a nucleotide sequence substantially complementary to the first substance, and the combination of the first substance selected from the group consisting of proteins, peptides, and substances binding to an antibody, and the second substance selected from the group consisting of proteins, peptides, and substances binding to an antibody, which specifically binds to the first substance.

The present invention enables immobilization of proteins, nucleic acids, or the like active substances on a carrier simply, efficiently, and strongly, which is important for detecting these active substances.

## DETAILED DESCRIPTION OF THE INVENTION

### 〈1〉 Carriers and Methods of Production Thereof

The carrier used in one embodiment of the present invention is the carrier for immobilizing a biologically active substance comprising a base material insoluble in solvents and having an isocyanate group on its surface. This carrier is hereinafter simply referred to as "the isocyanate carrier."

The carrier used in another embodiment of the present invention is the carrier for immobilizing a biologically active substance comprises a base material insoluble in solvents and having an carbodiimide group on its surface. This carrier is hereinafter simply referred to as "the carbodiimide carrier."

The compound having a carbodiimide group bound to the surface of the base material is sometimes simply referred to as "the carbodiimide compound."

The isocyanate carrier and the method of producing it, and the carbodiimide carrier and the method of producing it are illustrated below.

### A. Isocyanate Carriers and Methods of Production Thereof

#### (1) Base materials

A base material used for the carrier for immobilizing a biologically active substance according to the present invention plays a role of a support for the carrier and is insoluble in solvents. More specifically, an isocyanate group is introduced onto the surface of the base material used in the present invention as described below to serve as a carrier and a biologically active substance is immobilized thereon. The carrier with the active substance being immobilized thereon is used to produce or analyze physiologically active substances. The carrier is substantially insoluble in various solvents such as aqueous solvents and organic solvents used during the procedure of the above production or analysis of physiological substances. The base material used in the present invention is not particularly limited as long as it is insoluble in solvents as described above and basically solid or gel at the ordinary temperature or within the range of the ordinary temperature (0 to 100°C). Specific examples of the material for the base material of the carrier include plastics, inorganic polymers, metals, natural polymers, and ceramics.

Examples of plastics are polyethylene, polystyrene, polycarbonate, polypropylene, polyamide, phenol resin, epoxy resin, polycarbodiimide resin, poly(vinyl chloride), poly(vinylidene fluoride), poly(ethylene fluoride), polyimide, and acryl resin, etc.

Examples of inorganic polymers are glass, quartz, carbon, silica gel, graphite, etc.

Examples of metals are those which are solid at the ordinary temperature such as gold, platinum, silver, copper, iron, aluminum, magnet, paramagnet, apatite, etc.

Examples of natural polymers are cellulose, cellulose derivatives, chitin, chitosan, alginic acid, alginate, etc.

Examples of ceramics are alumina, silica, silicon carbide, silicon nitride, boron carbide, etc.

The above base materials are in the form of, for example, film, plate, particle, molded product (beads, strip, wells of a multiwell plate, tube, mesh, foam produced by continuous foaming, membrane, paper, needle, fiber, plate, slide, or a cell incubation container), or latex. As a matter of course, its size is not particularly limited.

(2) Production of Carriers

The carrier for immobilizing a biologically active substance of the present invention is the above-described base material insoluble in solvents and having an isocyanate group on its surface. This carrier of the present invention can be obtained by, for example, a method to directly introduce an isocyanate group for immobilizing a biologically active substance when used as a carrier onto the surface of the above base material by an appropriate means, a method to bind a compound in the form of film having an isocyanate group onto the surface of the above base material by coating or similar means, or a method to bind a compound having an isocyanate group on the surface of the above base material via a covalent bond.

More specifically, the method of binding a compound in the form of film having an isocyanate group onto the surface of the base material by coating or a similar method is carried out by, for example, dissolving a compound in the form of film having an isocyanate group in an appropriate solvent if necessary, coating the resulting solution on the whole or part of the surface of the base material by means of spraying, dipping, brushing, stamp, deposition, film coating, etc. And drying the product if required. Specific examples of the compound having an isocyanate group that can be coated on the surface of the base material by the above method include polycarbodiimide compounds having an isocyanate group at its end, trialkoxysilane having an isocyanate group such as isocyanate propyltriethoxysilane.

The compound having an isocyanate group can be bound onto the surface of the base material by, for example, covalently binding a compound having an isocyanate group and the other functional group that is capable of covalently binding to the surface of the base material, to a functional group present on the surface of the base material, which is capable of covalently binding to the above functional group of the compound, by an appropriate method. The carrier of the present invention obtained by binding a compound having an isocyanate group onto the surface of the base material insoluble in solvents via a covalent bond is excellent in durability since the compound having an isocyanate group is strongly bound onto the surface of the base material via a covalent bond.

Furthermore, the method of binding a compound having an isocyanate group onto the surface of the base material via a covalent bond is exemplified by the production method according to the present invention as described below.

The production method of the present invention is the method for producing a carrier for immobilizing a biologically active substance comprising a base material insoluble in solvents and having an isocyanate group on its surface, wherein said method comprises a step of covalently binding a compound having more than two isocyanate groups, more than one isocyanate groups and more than one functional groups other than the isocyanate group, or more than one isocyanate groups and a halogen atom (hereinafter sometimes simply referred to as "the isocyanate compound") onto a functional group on the surface of the base material insoluble in solvents, which is capable of covalently binding to the isocyanate group or to the functional group other than the isocyanate group or a halogen atom with leaving at least one isocyanate group of the compound free.

Examples of the compound having more than two isocyanate groups in its molecule used in the production method according to the present invention include hexamethylenediisocyanate, toluenediisocyanate, tetramethylxylenediisocyanate, naphthalenediisocyanate, etc.

Examples of the functional group other than the isocyanate group of the compound having more than one isocyanate groups and more than one functional groups other than the isocyanate group, or more than one isocyanate groups and a halogen atom in its molecule include a hydroxyl group, an amino group, an imino group, a carboxyl group, etc. Such an isocyanate compound is exemplified by chloromethyl isocyanate, chloroethyl isocyanate, etc.

The base material insoluble in solvents used in the production method according to the present invention, which has on its surface a functional group capable of covalently binding to an isocyanate group, or a functional group other than an isocyanate group or a halogen atom of the above compound includes those insoluble in solvents described in 〈1〉A(1) on the surface of which a functional group capable of covalently binding to the above-described groups is introduced. The functional group to be introduced is not particularly limited as long as it is capable of covalently binding to an isocyanate group, or a functional group other than an isocyanate group or a halogen atom of the above compound. Specific examples thereof include a hydroxyl group, an imino group, an amino group, a carboxyl group, etc. These functional groups are appropriately selected depending on the functional group of the above isocyanate compound and bound to the surface of the base material.

The method for introducing the above-described functional groups on the surface of the base material insoluble in solvents is appropriately selected depending on the material of the base material or the functional groups to be introduced. The functional groups can be introduced on the whole or part of the surface of the base material.

For example, an amino group can be introduced onto the whole of the surface of the glass base material by dissolving amino-substituted organoalkoxysilane such as 3-aminopropyltriethoxysilane in an appropriate solvent, dipping a glass base material in the resulting solution at about 70 to 80°C for about 2 to 3 hours, taking the base material out of the solution to wash it with water, and heat drying it at about 100 to 200°C for about 4 to 5 hours.

A functional group other than an amino group can be introduced onto the glass base material, or an amino group can be introduced onto the base material made of the material other than glass by a known method conventionally used for introducing various functional groups onto the surface of various materials as listed in the above description of the base material.

Some plastic base materials among the base materials listed in 〈1〉A(1) have the above functional groups on their surface originally. In this case, such base materials can be used as they are without introducing the functional groups on their surface. It is also possible to introduce the functional groups to such plastic base materials to be used in the present invention.

In the production method of the present invention, a compound having more than two isocyanate groups, more than one isocyanate groups and more than one functional groups other than the isocyanate group, or more than one isocyanate groups and a halogen atom is reacted with the base material insoluble in solvents, which has on its surface a functional group capable of covalently binding to an isocyanate group, or the above-described functional group other than an isocyanate group or a halogen atom, under appropriate conditions to covalently bind the above compound to the above functional group on the surface of the base material with leaving at least one isocyanate group of the above compound free. In other words, when the compound has more than one isocyanate groups and more than one functional groups other than an isocyanate group or more than one isocyanate groups and a halogen atom, the reaction is carried out under such conditions that the functional group other than an isocyanate group or a halogen atom is subjected to the covalent bond. When the compound having only an isocyanate group as a functional group is used, the reaction is carried out under such conditions that all of the isocyanate groups are not subjected to the covalent bond.

The thus-obtained carrier for immobilizing a biologically active substance according to the present invention comprising a base material insoluble in solvents and having an isocyanate group on its surface can be used to immobilize various active substances utilizing the reactivity of the isocyanate group. The isocyanate group is reactive, for example, with a hydroxyl group shown in the following formula (III) and with an amino group shown in the following formula (IV).

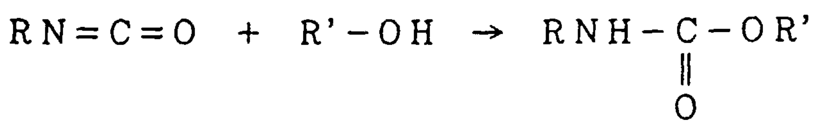

· · · (III)

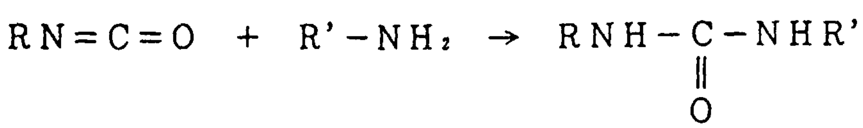

· · · (IV)

B. Carbodiimide Carriers and Methods for Production Thereof

(1) Base materials

A base material used for the carrier for immobilizing a biologically active substance according to the present invention plays a role of a support for the carrier and is insoluble in solvents. More specifically, a functional group is introduced onto the surface of the base material used in the present invention as described below if necessary and is subjected to a binding reaction with a compound having a carbodiimide group to serve as a carrier so that a biologically active substance is immobilized thereon. The carrier with the active substance being immobilized thereon is used to

produce or analyze physiologically active substances. The carrier is substantially insoluble in various solvents such as aqueous solvents and organic solvents used during the procedure of the above production or analysis of physiological substances. The base material used in the present invention is not particularly limited as long as it is insoluble in solvents as described above and basically solid or gel at the ordinary temperature or within the range of the temperature (0 to 100°C). Specific examples of the material for the base material of the carrier include plastics, inorganic polymers, metals, natural polymers, and ceramics. Examples of plastics, inorganic polymers, metals, natural polymers, and ceramics are the same as described in 〈1〉 A(1).

The above base materials are in the form of, for example, film, plate, particle, molded products (beads, strip, wells of a multiwell plate, tube, mesh, foam produced by continuous foaming, membrane, paper, needle, fiber, plate, slide, or a cell incubation container), or latex. As a matter of course, its size is not particularly limited.

(2) Compound having a Carbodiimide Group

The compound having a carbodiimide group which is covalently bound on the surface of the base material of the carrier of the present invention includes low molecular weight carbodiimides such as monocarbodiimide or dicarbodiimide, which are synthesized by the usually used methods for producing carbodiimides, such as dehydration of urea or polycarbodiimide or desulfurization of thiourea, produced by the method described in Japanese Patent Application Laid-open No Sho 51-61599, the method of L.M. Alberino et al. (J. Appl. Polym. Sci., 21, 190 (1990)), or the method described in Japanese Patent Application Laid-open No. Hei 2-292316.

The "compound having a carbodiimide group" used herein that means a compound having a carbodiimide group bound onto the surface of the base material via a covalent bond is defined as a compound excluding a group involved in the covalent bond between the compound and the surface of the base material. (Actually it is a "group," but is referred to as "compound" for convenience.) In other words, the compound having a carbodiimide group used herein means a compound without a functional group involved in the covalent bond between it and the surface of the base material.

The above-described polycarbodiimide can be produced in the presence of a catalyst that promotes the reaction converting an isocyanate group of an organic polyisocyanate compound to carbodiimide (e.g. 3-methyl-1-phenyl-2-phospholene-1-oxide).

Examples of the above organic polyisocyanate compound used for the production of polycarbodiimide include 4,4'-dicyclohexylmethanediisocyanate, m-tetramethylxylilenediisocyanate, 2,4-tolylenediisocyanate, 2,6-tolylene-diisocyanate, a mixture of 2,4-tolylenediisocyanate and 2,6-tolylenedi-isocyanate, crude tolylenediisocyanate, crude methylenediphenyldiisocyanate, 4,4',4''-triphenylmethylenetriisocyanate, xylenediisocyanate, hexamethylene-1,6-diisocyanate, lysine diisocyanate, hydrogenated methylenediphenyldi-isocyanate, m-phenyidiisocyanate, naphthylene-1,5-diisocyanate, 4,4'-biphenylenediisocyanate, 4,4'-diphenylmethanediisocyanate, 3,3'-dimethoxy-4,4'-biphenyldiisocyanate, 3,3'-dimethyldiphenylmethane-4,4'-diisocyanate, isopholonediisocyanate, and an optional mixture thereof.

The isocyanate group of the above polyisocyanate compounds or a mixture thereof are converted to carbodiimides by condensation. An appropriate amount of one or more than two monoisocyanate can be added at an appropriate timing to block the end of the carbodiimide compound to thereby adjust the molecular weight (degree of polymerization). Monoisocyanate can be added in an appropriate amount in advance before the condensation reaction. Examples of the monocyanate include phenylisocyanate (ortho, meta, para)-tolylisocyanate, dimethylphenylisocyanate, n-butylisocyanate, cyclohexylisocyanate, methylisocyanate, etc. The degree of polymerization can also be adjusted by the concentration of the polyisocyanate compound, the reaction time, and the like conditions.

The end blocking agent can also be a compound derived from an isocyanate compound that can be easily produced by the reaction between about 1 mole of a compound having an alkyl group with a functional group such as -OH, $-NH_2$, -COOH, -SH, -NH, etc. at its end and 2 moles of aromatic diisocyanate.

Various catalysts can be used as the above-described catalyst that promotes the conversion of the organic isocyanate to carbodiimide. In view of the yield and the like, suitable catalysts include 1-phenyl-2-phospholene-1-oxide, 3-methyl-1-phenyl-2-phospholene-1-oxide, 1-ethyl-2-phospholene-1-oxide, and their 3-phospholene isomers.

The above polycarbodiimide compounds are produced without a solvent or in a non-reactive organic solvent. One or a mixture of the thus-produced polycarbodiimide can be used as a carbodiimide compound in the present invention. These polycarbodiimide compounds may be partially crosslinked.

Other carbodiimide compounds, such as carbodiimide compounds to the molecules of which a polyoxyethylene chain is added to provide hydrophilicity on the compound as described in Japanese Patent Application Laid-open No. Sho 63-172718 or Japanese Patent Application Laid-open No. Sho 63-264128 can also be used for the carrier of the present invention. Furthermore, low molecular weight carbodiimide compounds such as monocarbodiimide compounds and dicarbodiimide compounds can be used for the carrier of the present invention.

Any of the above carbodiimide compounds bound to the surface of the base material of the carrier of the present invention via a covalent bond preferably has 5 to 30 carbodiimide groups in its molecule, more preferably 7 to

20 groups. When the number of the carbodiimide groups contained in the carbodiimide compound is more than 5 and less than 30, properties suitable for immobilizing a biologically active substance can be obtained and its solution has an appropriate viscosity to readily handle it.

As described above, the carbodiimide groups of the carbodiimide compounds are highly reactive and thus react with almost all active hydrogen atoms of alcohol, amine, thiol, phenol, carboxylic acid, and so on. Besides the reaction between the above-described carbodiimide derivative and carboxylic acid, the reaction with alcohol proceeds as shown in the following formula (V) and the reaction with an amino group proceeds as shown in the following formula (VI) (Frederik Kurzer, K. Douraghi-Zadeh, Chemical Reviews, 67, 117-135 (1967) and Andrew Williams, Ibrahim T. Ibrahim, Chemical Reviews, 81, 599-606 (1981)).

$$C_2H_5OH+C_6H_5N{=}C{=}NC_6H_5 \rightarrow C_6H_5NHC({=}NC_6H_5)OC_2H_5 \qquad (V)$$

$$RN{=}C{=}NR+R'NH_2 \rightarrow RNHC({=}NR')NHR \qquad (VI)$$

Therefore, a biologically active substance can be immobilized strongly on the carrier of the present invention through the carbodiimide compound utilizing the above reactivity of the carbodiimide group.

(3) Production of Carriers

The carrier for immobilizing a biologically active substance of the present invention is the above base material onto the surface of which the above-described compound having a carbodiimide group is bound via a covalent bond. This carrier of the present invention can be obtained by, for example, a method to directly introduce a carbodiimide group for immobilizing a biologically active substance when used as a carrier onto the surface of the above base material by an appropriate means, a method to bind a compound in the form of film having a carbodiimide group onto the surface of the above base material by coating or similar means, or a method to bind a compound having a carbodiimide group on the surface of the above-described base material via a covalent bond.

More specifically, the method of binding a compound in the form of a film having a carbodiimide group onto the surface of the base material by coating or a similar method is carried out by, for example, dissolving a compound in the form of a film having a carbodiimide group in an appropriate solvent if necessary, coating the resulting solution on the whole or part of the surface of the base material by means of spraying, dipping, brushing, stamp, deposition, film coating, etc, and drying the product if required.

Among the above-described methods, the carrier of the present invention is obtained preferably by covalently binding a carbodiimide compound having a carbodiimide group that is used to immobilized a biologically active substance and the other functional group that is capable of covalently binding to the surface of the base material to a functional group present on the surface of the base material, which is capable of covalently binding to the above functional group of the carbodiimide compound, by an appropriate method.

More specifically, the carrier of the present invention can be produced by the production method of the present invention as described below.

The production method of the present invention is the method for producing a carrier for immobilizing a biologically active substance comprising a base material insoluble in solvents and a carbodiimide compound having a carbodiimide group bound onto the surface of the base material via a covalent bond, wherein said method comprises a step of covalently binding a compound having more than two carbodiimide groups, more than one carbodiimide groups and more than one functional groups other than the carbodiimide group onto a functional group on the surface of the base material insoluble in solvents, which is capable of covalently binding to the carbodiimide group or to the functional group other than the carbodiimide group with leaving at least one carbodiimide group of the compound free.

The compound used in the above production method of the present invention has more than two carbodiimide groups or more than one carbodiimide groups and more than one functional groups other than a carbodiimide group.

Examples thereof are the carbodiimide compounds having more than two carbodiimide groups and the carbodiimide compounds having more than one carbodiimide groups and more than one functional groups other than the carbodiimide group as described in 〈1〉 B(2). In the production method of the present invention, it is also possible to use the carbodiimide compounds as recited in 〈1〉 B(2) to which a functional group to be involved in a covalent bond selected from the group consisting of a hydroxyl group, an imino group, an amino group, a carboxyl group, an isocyanate group, and an isothiocyanate group is introduced by an appropriate method. Furthermore, the carbodiimide compounds recited in 〈1〉 B(2) into which an additional carbodiimide group is introduced as a functional group to be involved in a covalent bond can also be used in the production method of the present invention. The above-described functional groups can be introduced into the carbodiimide compound by the known methods.

The base material insoluble in solvents used in the production method according to the present invention,

which has on its surface a functional group capable of covalently binding to a carbodiimide group, or a functional group other than a carbodiimide group of the above compound includes those insoluble base materials in solvents described in 〈1〉B(1) on the surface of which a functional group capable of covalently binding to the above-described groups is introduced. The functional group to be introduced is not particularly limited as long as it is capable of covalently binding to a carbodiimide group, or a functional group other than a carbodiimide group of the above compound. Specific examples thereof include a hydroxyl group, an imino group, an amino group, a carboxyl group, a carbodiimide group, etc. These functional groups are appropriately selected depending on the functional group of the above carbodiimide compound to be involved in the covalent bond and bound to the surface of the base material.

The method for introducing the above-described functional groups on the surface of the base material insoluble in solvents is appropriately selected depending on the material of the base material or the functional groups to be introduced. The functional groups can be introduced on the whole or part of the surface of the base material.

For example, an amino group can be introduced onto the whole of the surface of the glass base material by dissolving amino-substituted organoalkoxysilane such as 3-aminopropyltriethoxysilane in an appropriate solvent, dipping a glass base material in the resulting solution at about 70 to 80°C for about 2 to 3 hours, taking the base material out of the solution to wash it with water, and drying it under heating at about 100 to 200°C for about 4 to 5 hours.

A functional group other than an amino group can be introduced onto the glass base material, or an amino group can be introduced onto the base material made of the material other than glass by a known method conventionally used for introducing various functional groups onto the surface of various materials as listed in the above description of the base materials.

Some plastic base materials among the base materials listed in 〈1〉B(1) have the above functional groups on their surface originally. In this case, such base materials can be used as they are without introducing the functional groups on their surface. It is also possible to introduce the functional groups to such plastic base materials to be used in the present invention.

In the production method of the present invention, a compound having more than two carbodiimide groups, more than one carbodiimide groups and more than one functional groups other than the carbodiimide group is reacted with the base material insoluble in solvents, which has on its surface a functional group capable of covalently binding to a carbodiimide group, or the above-described functional group other than a carbodiimide group under appropriate conditions to covalently bind the above compound to the above functional group on the surface of the base material with leaving at least one carbodiimide group of the above compound free. In other words, when the compound has more than one carbodiimide groups and more than one functional groups other than a carbodiimide group, the reaction is carried out under such reaction conditions that the functional group other than a carbodiimide group is subjected to the covalent bond. When the compound having only a carbodiimide group as a functional group is used, the reaction is carried out under such conditions that all of the carbodiimide groups are not subjected to the covalent bond.

The thus-obtained carrier for immobilizing a biologically active substance according to the present invention can be used to immobilize various active substances utilizing the reactivity of the carbodiimide compound.

〈2〉Biologically Active Substances and Their Immobilization on the Carrier

The following immobilization can be applied to both the isocyanate carrier described in 〈1〉A and the carbodiimide carrier described in 〈1〉B, unless otherwise specified.

(1) Biologically Active Substances

When the isocyanate carrier described in 〈1〉A is used as the carrier of the present invention, a biologically active substance to be immobilized on the carrier is not particularly limited as long as it has a functional group capable of covalently binding to the isocyanate group. These substances are biopolymers such as proteins, peptides, other substances binding to antibodies, and nucleic acid.

When the carbodiimide carrier described in 〈1〉B is used as the carrier of the present invention, a biologically active substance to be immobilized on the carrier is not particularly limited as long as it has a functional group capable of covalently binding to the carbodiimide group. These substances are biopolymers such as proteins, peptides, other substances binding to antibodies, and nucleic acid.

Specific examples thereof include proteins and peptides such as protein hormones and peptide hormones, e.g., insulin, adrenocorticotrophic hormone (ACTH), oxytocin, etc. Enzymes such as choline esterase, amylase, pepsin, etc. or their precursors, protein antigens such as HBs antigen, and HIV antigen, proteins that binds to antibodies, such as protein A, substances that bind to antibodies such as hapten with a low molecular weight, nucleic acids such as natural or synthetic DNA (including oligonucleotides) and RNA (including oligonucleotides).

The following substances can also be used as the biologically active substances used in the present invention: physiologically active substances with antibiotic activity such as penicillin, ampicillin, cephalosporin, kanamycin,

streptomycin, fradinomycin, destomycin, kasugamycin, tylosin, erythromycin, oleadomycin, spiramycin, lincomycin, colistin, bacitracin, salinomycin, monensin, Lasalocid, tetracycline and its analogues, chloramphenicol, virginiamycin, etc.; synthetic antibiotics such as sulfa drug, oxophosphoric acid, pyromidic acid, furazolidone, difurazone, etc.; natural toxins such as aflatoxin, T2 toxin, zearalenone, deoxynivalenol, patulin, fumonisin, HT-2, okratoxin, tetrodotoxin, okadic acid, saxitoxin, goniotoxin, botulinum toxin, etc.; synthetic chemicals such as agricultural chemicals, e.g., dioxin, 2,4-D, benomyl, aldicarb, carbofuran, methomyl, DDVP, malathon, paraquat, diazinon, phenitrothione, endrin, aldrin, heptachlor, etc.; and biomolecules such as hemoglobin, alpha-fetoprotein, immunoglobulin, albumin, antithrombin, thrombin, plasminogen, ferritin, thyroglobulin, gelatin, cholesterol, testosterone, corticosterone, progesterone, ergosterol, estradiol, cytochrome C, adrenaline, various vitamins, etc.

Also usable are antibodies that bind to the above proteins, peptides, or other biologically active substances. Such antibodies can be obtained by immunizing mammals such as rats, guinea pigs, rabbits, mice, goats, sheep, horses, and cattle with a binding product of the above substance and the carrier for immunization. Monoclonal antibodies can be obtained by immunizing mice with the above substance, and allowing hybridomas that are obtained from the lymphocytes of the immunized mice and mouse myeloma cells to produce the antibodies.

(2) Immobilization on the Carrier

When the isocyanate carrier described in 〈1〉 A is used as the carrier for immobilizing a biologically active substance according to the present invention comprising a base material insoluble in solvents and having an isocyanate group on its surface, the biologically active substance can be immobilized on the carrier by contacting the carrier with the biologically active substance under the conditions that the isocyanate group of the carrier reacts with the biologically active substance. Specifically, it is preferable that the contact is performed generally in water or buffer so as to maintain the activity of the biologically active substance to be immobilized. The temperature during the contact is preferably about 0 to 100°C so as to maintain the activity of the biologically active substance to be immobilized. The optimum conditions can be appropriately selected depending on the kinds of the biologically active substance to be immobilized.

When the carbodiimide carrier described in 〈1〉 B is used as the carrier for immobilizing a biologically active substance according to the present invention comprising a base material insoluble in solvents on the surface of which a compound having a carbodiimide group is bound via a covalent bond, the biologically active substance can be immobilized on the carrier by contacting the carrier with the biologically active substance under the conditions that the carbodiimide group of the carrier reacts with the biologically active substance. Specifically, it is preferable that the contact is performed generally in water or buffer so as to maintain the activity of the biologically active substance to be immobilized. The temperature during the contact is preferably about 0 to 100°C so as to maintain the activity of the biologically active substance to be immobilized. The optimum conditions can be appropriately selected depending on the kinds of the biologically active substance to be immobilized.

The thus-obtained immobilized biologically active substance is strongly bound to the carrier and does not detach even after it is washed in the manner usually used in the field of immunoassay (washing with a surface active agent). It can thus be widely applied to immobilized enzymes used in the industry of physiological chemistry, immobilized antibody or antigen for immunological use, and immobilized nucleic acid as diagnostic agents.

(3) Assay of Biologically Active Substances

The carrier of the present invention specifically exhibits its effect in the following assay.

When the isocyanate carrier described in 〈1〉 A is used as the carrier for immobilizing a biologically active substance according to the present invention comprising a base material insoluble in solvents and having an isocyanate group on its surface, a first biologically active substance or a second substance in a sample can be assayed by reacting the first substance immobilized on the carrier with the second substance that may be specifically bound to the first substance, and detecting the second substance that is indirectly bound to the carrier by the bond between it and the first substance or the second substance that is not bound to the carrier. In this case, the first substance is immobilized on the carrier by the isocyanate group. Examples of the first biologically active substances are exactly the same as those described in 〈2〉 (1).

When the carbodiimide carrier described in 〈1〉 B is used as the carrier for immobilizing a biologically active substance according to the present invention comprising a base material insoluble in solvents on the surface of which a compound having a carbodiimide group is bound via a covalent bond, a first biologically active substance or a second substance in a sample can be assayed by reacting the first substance immobilized on the carrier with the second substance that can be specifically bound to the first substance, and detecting the second substance that is indirectly bound to the carrier by the bond between it and the first substance or the second substance that is not bound to the carrier. In this case, the first substance is immobilized on the carrier by the carbodiimide group. Examples of the first biologically active substances are exactly the same as those described in 〈2〉 (1).

On the other hand, the second biologically active substances are, like the first substance, proteins, peptides, antigenic substance, nucleic acid, and other physiologically active substances, and specifically bind to the first substance. For example, when one of the first substance and the second substance is a protein, nucleic acid, or the other physiologically active substance, the other is an antibody against it. When the one is nucleic acid, the other is that having a nucleotide sequence substantially complementary to the nucleotide sequence of the nucleic acid. The term "substantially complementary" used herein means the sequence can hybridize with another sequence via a hydrogen bond to form a double strand even if there are one or more than two mismatches.

The substance to be analyzed may be either the first substance or the second substance.

The method of assaying a biologically active substance according to the present invention can be effected by binding the above first biologically active substance to the carrier, reacting the carrier with the second substance, and detecting the second substance that is indirectly bound to the carrier by the bond between it and the first substance or the second substance that is not bound to the carrier.

When the isocyanate carrier described in 〈1〉 A is used, the first substance can be immobilized on the carrier by contacting the carrier with the active substance. The isocyanate group on the surface of the base material of the carrier reacts with a hydroxyl group, an amino group, a thiol group, a carboxyl group, etc. of the first substance, the first substance covalently binds to the isocyanate compound. The first substance is thus immobilized on the carrier.

The first substance can be contacted with the carrier in the same manner as described in 〈2〉 (2) preferably in water or buffer so as to maintain the biological activity of the first substance. The temperature during the contact is preferably from 0 to 100°C so as to maintain the activity of the substance.

In order to prevent the second substance etc. from non-specifically binding to the carrier, free isocyanate groups remaining after the first substance is immobilized on the carrier, are preferably blocked by contacting an excessive amount of bovine serum albumin (BSA), casein, salmon sperm DNA, and so on with the carrier.

When the carbodiimide carrier described in 〈1〉 A is used, the first substance can be immobilized on the carrier by contacting the carrier with the active substance. The carbodiimide group of the carbodiimide compound covalently bound onto the surface of the base material that serves as a support of the carrier reacts with a hydroxyl group, an amino group, a thiol group, a carboxyl group, etc. of the first substance, the first substance covalently binds to the carbodiimide compound. The first substance is thus immobilized on the carrier.

The first substance can be contacted with the carrier in the same manner as described in 〈2〉 (2) preferably in water or buffer so as to maintain the biological activity of the first substance. The temperature during the contact is preferably from 0 to 100°C so as to maintain the activity of the substance.

In order to prevent the second substance or the like from non-specifically binding to the carrier, free carbodiimide groups remaining after the first substance is immobilized on the carrier are preferably blocked by contacting an excessive amount of bovine serum albumin (BSA), casein, salmon sperm DNA, and so on with the carrier.

After the first substance immobilized on the carrier is reacted with the second substance, the second substance bound to the carrier can be detected by solid phase immunoassay, the nucleic acid hybridization method, or the similar usually used methods. For example, when the first substance is to be assayed, the immobilized first substance is reacted with the second substance labeled with a label, and the second substance is detected or quantified by detecting or quantifying the label immobilized on the carrier. The first substance is thus detected and quantified. Alternatively, the second substance that is not bound to the first substance can be detected or quantified in place of assaying the bound second substance.

When the second substance is to be assayed, the reaction between the first substance and the second substance is carried out in the reaction system containing the second substance labeled with a label, the amount of the labeled second substance bound to the first substance is determined to thereby indirectly quantify the amount of the second substance in the sample (inhibition assay).

The second substance bound to the carrier can be also detected by reacting it with a third substance that specifically binds to the second substance. For example, when the second substance is an antigen, and the first substance is a polyclonal or monoclonal antibody (the first antibody) against the antigen, the second substance can be detected by reacting the carrier-antibody-antigen complex with a polyclonal antibody or another monoclonal antibody having an epitope different from that of the above monoclonal antibody (the second antibody) to form a carrier-antibody-antigen-antibody complex, and detecting the third substance in the resulting complex (sandwich assay). In this occasion, when the third substance is labeled, the label is detected. When the third substance is not labeled, another labeled substance that binds to the third substance can be used. For example, in the above case, the first antibody and the second antibody are prepared using different animals, an antibody against immunoglobulin from the animal used for the preparation of the second antibody is used as the third substance. Likewise, when nucleic acid is to be assayed, the first nucleic acid immobilized on the carrier is bound to the second nucleic acid that specifically binds thereto, the binding product is further bound to the third nucleic acid that specifically binds to the second substance, not to the first substance, and the amount of the third nucleic acid bound to the carrier is determined to quantify the second nucleic acid.

Alternatively, the second substance can be detected using a carrier in the emulsified form by the usual

agglutination method.

Examples of the labels include radioactive substances, fluorescent substances, enzymes, dyes, chemoluminescent substances, digoxygenin, etc. When radioactive substances, fluorescent substances, or enzymes are used as a label, the label can be detected by using a scintillation counter, by exposing it to a film, or directly by observation with the naked eyes. When enzymes are used, a dye substrate that develops color or emits the light through the enzymatic reaction is used, and the color or the light is detected. Examples of the enzymes are peroxidase, β-D-galactosidase, alkaline phosphatase, lysozyme, and the other usually used enzymes.

The label itself is not necessarily detectable. For example, when biotin is used as a label, it can be indirectly detected using an enzyme bound to avidin or streptoavidin that specifically binds to biotin.

After the reaction between the first substance and the second substance, and, if necessary, the third substance or another substance, the unreacted substances can be removed, or B/F separation can be carried out, in the same manner as in usually used solid phase immunoassay or the hybridization method. When the carrier is in the form of a container, a wash solution is added to the carrier, then discarded. This procedure is repeated several times. When the carrier is in the form of particles, the procedure that the carrier is suspended in the wash solution is repeated.

## EXAMPLES

Examples of the present invention is illustrated below.

## EXAMPLE 1

### Detection of DNA immobilized on isocyanated slide glass using labeled DNA

(1) Preparation of aminated slide glass

A solution of 10% (v/v) 3-aminopropyltriethoxysilane in ethanol (20 ml) was added to 180 ml of distilled water, and the mixture was stirred well. After the pH was adjusted to 3 to 4 by adding 6N HCl thereto, 15 pieces of slide glasses were dipped in the solution and treated under heating at 75°C for 2 hours. After completion of the heat treatment, slide glasses were taken out from the solution, washed well with distilled water, and dried by heating 115°C for 4 hours to obtain aminated slide glasses.

(2) Preparation of isocyanated slide glasses

Fifteen pieces of the aminated slide glasses obtained above were dipped in a 2.5% solution of hexamethylenediisocyanate in chloroform and immediately taken out. The glasses were washed twice with 200 ml of chloroform for 10 minutes and dried at 40°C for 2 hours to obtain isocyanated slide glasses.

(3) Immobilization of DNA oligomer on the isocyanated slide glasses

A capture oligonucleotide and biotinated oligonucleotide were synthesized with a DNA synthesizer. Biotin was introduced at the 5' end of the DNA oligomer using biotin phosphoramidite to serve as a probe.

A 1 μl portion of the dilution series of a capture solution (1 pmol/μl, 100 fmol/μl, 10 fmol/μl, and 1 fmol/μl) was dotted on the isocyanated slide glasses obtained above, incubated at 37°C for 15 minutes for immobilization, washed with water, and dried.

On the other hand, as a control, an oligomer that is not complementary to the above probe was immobilized in the same manner.

(4) Hybridization

A prehybridization solution (50 μl) was sprinkled on the isocyanated slide glasses on which the capture oligonucleotide or the control DNA oligomer was immobilized. The glasses were covered with parafilm and heated at 42°C for 30 minutes in an incubator. The prehybridization solution contained 5 x SSC (0.75 M NaCl and 0.075 M sodium citrate), 5 x Denhardt's solution [0.02% Ficoll, 0.02% BSA fraction V, and 0.02% poly(vinyl pyrrolidone)], 25 mM sodium phosphate (pH 6.6), 50% formamide, and 0.5 mg/ml denatured salmon sperm DNA.

Next, the parafilm was removed, and the prehybridization solution was slightly sucked up. A hybridization solution (50 μl) containing the probe was sprinkled on the glasses, which was then covered with parafilm and heated overnight in an incubator maintained at 42°C. The hybridization solution contained 1 pmol of the probe, 5 x SSC, 1 x Denhardt's solution, 25 mM sodium phosphate (pH 6.6), 45% formamide, 0.2 mg/ml denatured salmon sperm DNA,

and 10% dextran sulfate.

After hybridization, the parafilm was removed, and the hybridization solution was slightly sucked up. The posthybridization washing was carried out under the following three-step conditions to remove the probe non-specifically absorbed.

〈Posthybridization washing conditions〉

First step: 2 x SSC, 1% SDS; room temperature, 5 minutes, twice.
Second step: 0.2 x SSC, 1% SDS; 42°C, 5 minutes, twice.
Third step: 2 x SSC; room temperature, 5 minutes, once.

(5) Detection

After the posthybridization washing, the slide glasses were dipped in 50 ml of buffer A [0.2 M sodium chloride, 0.1 M Tris hydrochloride (pH 7.5), and 0.05% Triton X-100] supplemented with 3% BSA at room temperature for 30 minutes for blocking. The glasses were then dipped in 50 ml of streptoavidin-alkaline phosphatase conjugate solution (a 5000-fold dilution of the original solution with buffer A) to react at room temperature for 30 minutes. The glasses were then dipped in 50 ml of buffer A and allowed to stand at room temperature for 5 minutes. This procedure was repeated twice to remove the conjugate that was not bound to biotin.

The Buffer A was replaced once with 50 ml of buffer B (0.1 M sodium chloride / 0.1 M Tris hydrochloride, pH 9.5 / 50 ml of magnesium chloride). Finally, the glasses were dipped in a substrate solution (50 ml of buffer B + 17.5 μl of BCIP solution (50 mg of 5-bromo-4-chloro-3-indolylphosphate / 900 ml of dimethylformamide) + 35 μl of NBT solution (50 mg of nitro blue tetrazolium / 1.8 ml of 70% ethanol) and allowed to stand at room temperature for 3 hours to perform the color reaction. Signals were found for only the capture oligonucleotide having a complementary sequence within the range of 1 pmol/μl to 10 fmol/μl.

<u>Example 2</u>

The isocyanate slide glasses were prepared in the same manner as in Example 1 except for using toluenediisocyanate in place of hexamethylenediisocyanate. Like in Example 1, signals were found for only the capture oligonucleotide having a complementary sequence within the range of 1 pmol/μl to 10 fmol/μl.

<u>Example 3</u>

The isocyanate slide glasses were prepared in the same manner as in Example 1 except for using tetramethylxylenediisocyanate in place of hexamethylenediisocyanate. Like in Example 1, signals were found for only the capture oligonucleotide having a complementary sequence within the range of 1 pmol/μl to 10 fmol/μl.

<u>Example 4</u>

The isocyanate slide glasses were prepared in the same manner as in Example 1 except for using naphthalenediisocyanate in place of hexamethylenediisocyanate. Like in Example 1, signals were found for only the capture oligonucleotide having a complementary sequence within the range of 1 pmol/μl to 10 fmol/μl.

<u>Example 5</u>

<u>Detection of DNA immobilized on carbodiimidated slide glass using labeled DNA</u>

(1) Preparation of carbodiimidated slide glass

4,4'-dicyclohexylmethanediisocyanate (117.9 g) was reacted with 12.5 g of cyclohexylisocyanate in the presence of 1.3 g of a carbodiimide catalyst (3-methyl-1-phenyl-2-phospholene-1-oxide) at 180°C for 4 days under nitrogen atmosphere to obtain a carbodiimide compound (degree of polymerization, 10; number average molecular weight, 2400) that is in the form of powder at room temperature. A 10 g portion of the compound was dissolved in 200 ml of dichloromethane to obtain a solution of the carbodiimide compound.

Fifteen pieces of the aminated slide glasses obtained in the same manner as in Example 1 (1) were dipped

in 200 ml of the above solution of the carbodiimide compound, immediately taken out, and dried by heating at 60°C for 1 hour. The glasses were then washed twice with 200 ml of dichloromethane for 10 minutes, and dried at 40°C for 2 hours to obtain carbodiimidated slide glasses.

(2) Immobilization of DNA on the carbodiimidated slide glasses and detection of the DNA

The same procedure of Example 1 (3) to (5) was carried out except for using the above-obtained carbodiimidated slide glasses in place of the isocyanated slide glasses. Signals were found for only the capture oligonucleotide having a complementary sequence within the range of 1 pmol/μl to 100 fmol/μl.

Comparative Example 1

The same procedure of the above Examples was carried out except for dipping slide glasses directly in the solution of the carbodiimide solution without aminating slide glasses. The results showed an increase in the background level.

**Claims**

1. A carrier for immobilizing a biologically active substance comprising a base material insoluble in solvents and a compound having an isocyanate group bound to the surface of the base material via a covalent bond.

2. A carrier for immobilizing a biologically active substance comprising a base material insoluble in solvents and a compound having a carbodiimide group bound to the surface of the base material via a covalent bond.

3. The carrier according to claim 1 or 2, said base material is made of a material selected from the group consisting of plastics, inorganic polymers, metals, natural polymers, and ceramics.

4. A method of producing a carrier for immobilizing a biologically active substance comprising a base material insoluble in solvents and having an isocyanate group on its surface,
   wherein said method comprises covalently binding a compound having (i) more than two isocyanate groups, (ii) more than one isocyanate groups and more than one functional groups other than an isocyanate group or (iii) more than one isocyanate groups and a halogen atom, to a functional group on the surface of the base material insoluble in solvents with leaving at least one free isocyanate group of said compound, said functional group is capable of covalently binding to an isocyanate group, a functional group other than an isocyanate group, or a halogen atom.

5. A method of producing a carrier for immobilizing a biologically active substance comprising a base material insoluble in solvents and a compound having a carbodiimide group bound onto the surface of the base material via a covalent bond,
   wherein said method comprises covalently binding a compound having (i) more than two carbodiimide groups or (ii) more than one carbodiimide groups and more than one functional groups other than a carbodiimide group, to a functional group on the surface of the base material insoluble in solvents with leaving at least one free carbodiimide group of said compound, said functional group is capable of covalently binding to a carbodiimide group or a functional group other than a carbodiimide group.

6. A method of immobilizing a biologically active substance, wherein said method comprises;

   contacting a biologically active substance that is reactive with an isocyanate group or a carbodiimide group with a carrier for immobilizing a biologically active substance comprising a base material insoluble in solvents and a compound having an isocyanate group or a carbodiimide group bound onto the surface of the base material via a covalent bond.

7. The method according to claim 6, wherein said biologically active substance is selected from the group consisting of proteins, peptides, other substances that bind to antibodies, and nucleic acid.

8. A method for assaying a biologically active substance, said method comprising;

   reacting a first substance which is biologically active and immobilized on a carrier with a second substance

capable of specifically binding to the first substance,

and detecting the second substance which is indirectly bound or not bound to the carrier via a bond between itself, to analyze the first substance or the second substance in a sample,

wherein said carrier is the one for immobilizing a biologically active substance comprising a base material insoluble in solvents and a compound having an isocyanate group or a carbodiimide group bound onto the surface of the base material via a covalent bond, and said first substance is immobilized on the carrier via the isocyanate group or the carbodiimide group.

**9.** The method according to claim 8, wherein said first substance is nucleic acid, and said second substance is another nucleic acid having a nucleotide sequence substantially complementary to the sequence of the first nucleic acid.

**10.** The method according to claim 8, wherein said first substance is selected from the group consisting of proteins, peptides, and substances binding to an antibody, and said second substance is selected from the groups consisting of proteins, peptides, and substances binding to an antibody, which specifically binds to the first substance.